# EUROPEAN PATENT APPLICATION

(11) **EP 3 854 432 A1**
(43) Date of publication of application: **28.07.2021**
(21) Application number: 20153930.1
(22) Date of filing: 27.01.2020
(51) Int. Cl.: A61M 5/14, A61M 5/142, A61M 5/145

(54) **PUMP FOR DELIVERING LIQUID TO BE ADMINISTERED INTRAVENOUSLY**

(71) Applicant: Ivy Medical Holding B.V., 9747 AN Groningen (NL)
(72) Inventor: TUIN, Peter, 9747 AN Groningen (NL)
(74) Representative: V.O.

(57) **Abstract**

Infusion delivery pump with a supply conduit communicating with a pump chamber via a pump valve, while a delivery conduit is shut off from the pump chamber, during infusion intake. The delivery conduit communicates with the pump chamber, while the supply conduit is shut off from the pump chamber, during infusion delivery. A pump valve actuator and a pump drive are controlled by a control unit. A plunger drive, the pump valve actuator and the control unit are part of a base unit. A pump housing, a plunger, the supply conduit, the delivery conduit and a pump valve are part of a disposable unit removably attached to the base unit. The coupling of the pump valve to the pump valve actuator and the coupling of the plunger to the plunger drive are releasable.

## Description

### FIELD AND BACKGROUND OF THE INVENTION

The invention relates to a pump for delivering an intravenous ("IV") solution or other liquid, such as a medicament, to be administered intravenously.

Intravenous administration of a liquid, usually a solution, is commonly carried out by delivery from an IV bag using pressure resulting from gravity. To create and maintain pressure resulting from gravity, the IV bag is suspended at a level higher than the level range of the patient body, for instance from a pole projecting from a foot or mounted to a bed. Such a pole severely restricts the freedom of movement of the patient. IV administration of medication may be required for prolonged periods of time. Restricting mobility of a patient for a prolonged period of time is inconvenient for the patient, increases the need of nursery care and is particularly disadvantageous since it has been found that physical exercise and mobility has a positive effect on patient health, so that restricting patient mobility has an adverse effect on patient health and tends to lead to longer stays in hospital.

Delivery of an IV solution may be supported by a peristaltic or piston driven IV pump. Use of such pumps is generally indicated if a specific amount of a pharmacologic agent is to be administered and/or fluid overload is to be prevented. However, such pumps are not suitable to be worn on the patient body due to weight, size and certified use restrictions and usually mounted to an IV pole, so that no improvement regarding patient mobility is achieved. Furthermore, such IV pumps are costly.

### SUMMARY OF THE INVENTION

It is an object of the invention to provide a pump for providing liquid to be delivered intravenously that is wearable and can be used safely, easily and economically in a health care environment. More in particular, it is an object to provide a pump that is compatible with conventional IV bags and which accurately delivers IV liquid at a predetermined flow rate, regardless the orientation of the pump.

According to the invention, this object is achieved by providing a device according to claim 1. The invention can also be embodied in a disposable unit according to claim 15 for use in a base unit of such a pump.

In use of such a device, the parts of the pump that are in direct contact with infusion liquid, in particular the pump cylinders and the pump valve, and which need to be sterile at least when perfusion is started, to be replaceable as a single disposable unit, which is replaceable very easily. The base unit of the pump, in particular the relatively costly pump drive and the valve actuators can be re-used many times, so that costs are saved and relatively little waste is produced.

Particular elaborations and embodiments of the invention are set forth in the dependent claims. Features of the dependent claims applying to the disposable unit can also be applied advantageously in a disposable unit according to claim 15.

Further features, effects and details of the invention appear from the detailed description and the drawings.

### BRIEF DESCRIPTION OF THE DRAWINGS

Fig. 1 is a perspective view of a first example of an IV pump according to the invention;
Fig. 2 is a perspective view of the IV pump shown in Fig. 1 with a lid opened;
Fig. 3 is a perspective exploded view of the IV pump as shown in Fig. 2 without an exchangeable and disposable cylinder and valve arrangement;
Fig. 4 is a cross-sectional view along a plane IV-IV in Fig. 5 of the exchangeable and disposable cylinder and valve arrangement;
Fig. 5 is a cross-sectional view along a plane V-V in Fig. 4 of the exchangeable and disposable cylinder and valve arrangement in a first operating condition;
Fig. 6 is a cross-sectional view along a plane V-V in Fig. 4 of the exchangeable and disposable cylinder and valve arrangement in a second operating condition;
Fig. 7 is a perspective view of a second example of an IV pump according to the invention;
Fig. 8 is a perspective view of the IV pump shown in Fig. 7 with a lid opened;
Fig. 9 is a top-plan view of the pump shown in Figs. 7 and 8 with an exchangeable and disposable cylinder and valve arrangement in a first operating condition;
Fig. 10 is a top-plan view of the pump shown in Figs. 7-9 with the exchangeable and disposable cylinder and valve arrangement in a second operating condition; and
Fig. 11 is a perspective view of the IV pump as shown in Fig. 8 without the exchangeable and disposable cylinder and valve arrangement.

### DETAILED DESCRIPTION

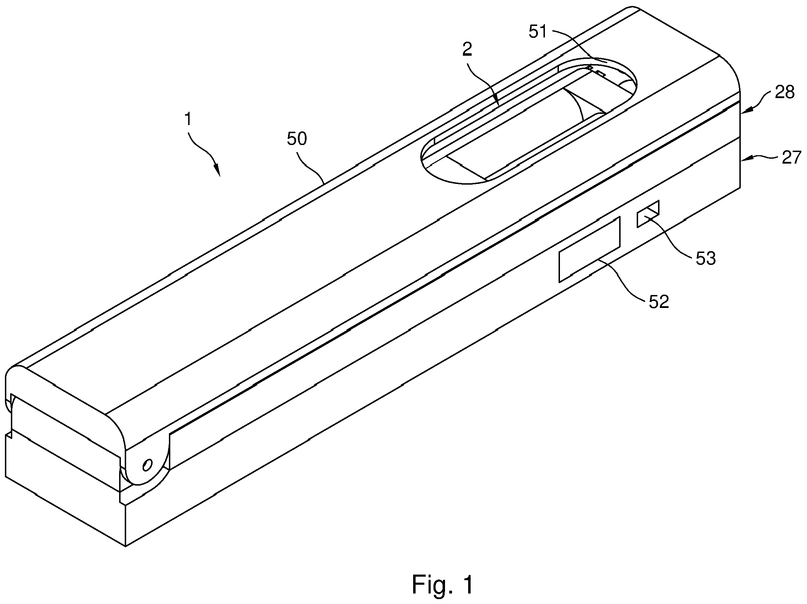

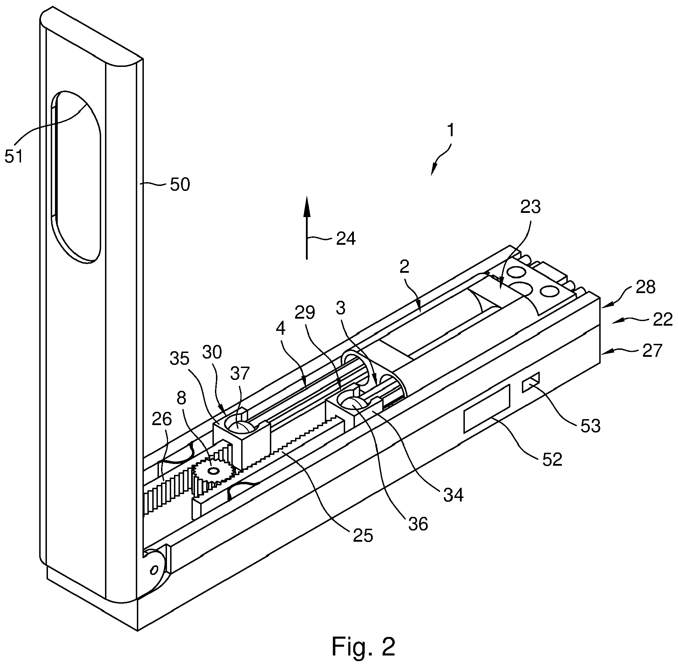

In Figs. 1-6, a first example of an infusion delivery pump 1 according to the invention is shown. The pump 1 has a pump housing 2 and two plungers 3, 4. The pump housing 2 and the plungers 3, 4 bound pump chamber 5 and, respectively 6. The plungers 3, 4 are reciprocally movable inwardly and outwardly of the pump housing 2 thereby increasing and decreasing the volume of the pump chambers 5 and, respectively, 6.

For driving displacement of the plungers relative 3, 4 to the pump housing 2, a plunger drive 7 is provided. The plunger drive 7 has a pinion wheel 8 in driving engagement with toothed racks 25, 26 which are coupled to respective ones of the plungers 3, 4. The plunger drive 7 is further composed of a shaft 9 engaging a coupling member 10 of an electric motor 11.

The pump 1 further has two supply conduit sections 12, 13 merging into a common supply conduit section 14 which communicates with one of the pump chambers 5, 6 for delivering infusion liquid into that pump chamber 5 or 6.

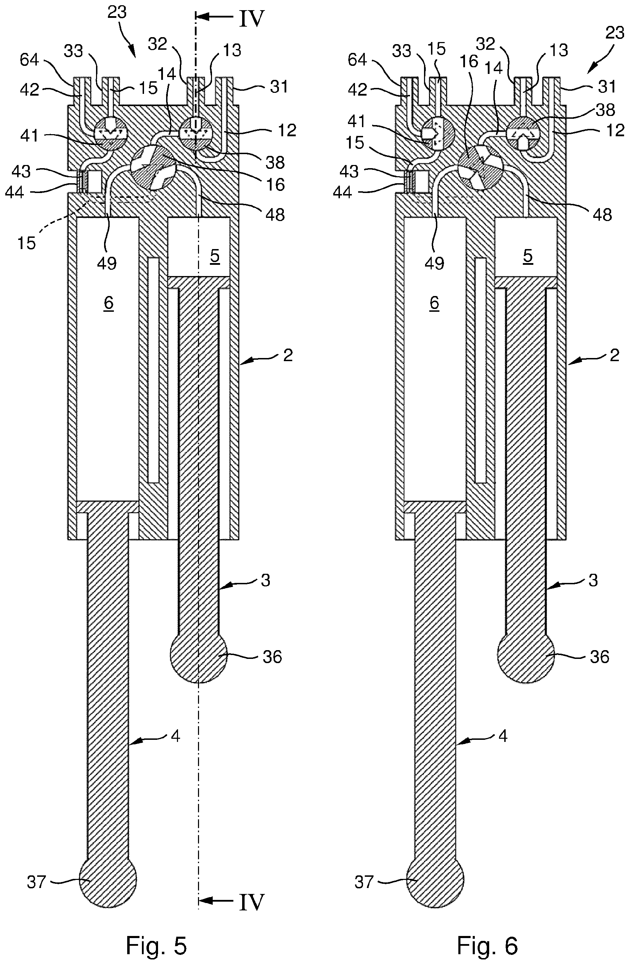

In the operating condition shown in Fig. 5, a delivery conduit 15 communicates with the other one of the pump chambers 5 or 6 for receiving and delivering infusion liquid from that pump chamber 5, 6.

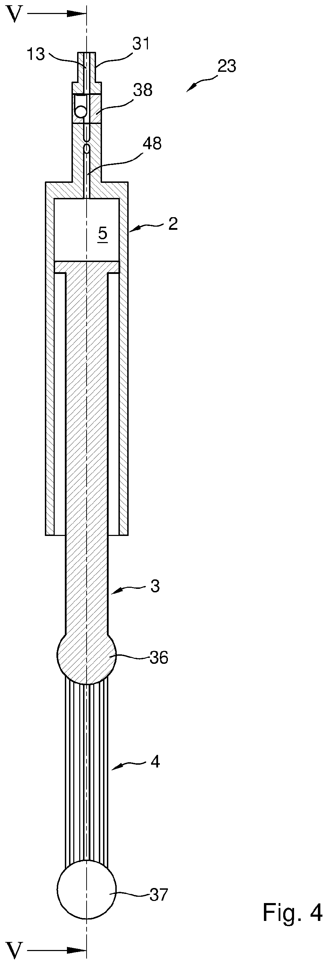

The supply conduit sections 12 or 13 and 14 communicate with one of the pump chambers via pump valve 16, while the delivery conduit 15 is shut off from that one of the pump chambers 5, if the pump valve 16 is in an infusion intake condition with regard to that pump chamber 5 as is shown in Figs. 4 and 5. If the pump valve 16 is in an infusion delivery condition with regard to the pump chamber 5, as is shown in Fig. 6, the delivery conduit 15 communicates with the pump chamber 5, while the supply conduit sections 12-14 are shut off from that pump chamber 5. For operating the pump valve 16, a pump valve actuator 17 is provided. The pump valve actuator 17 has a pump valve coupling member 18 for coupling the pump valve actuator 17 to the pump valve 16, an electric pump valve motor 20 and a pump valve motor coupling member 19 coupling the pump valve coupling member 18 to the pump valve motor 20.

A control unit 21 is arranged and connected for controlling the motor 11 of the plunger drive 7 and motor 20 of the pump valve actuator 17.

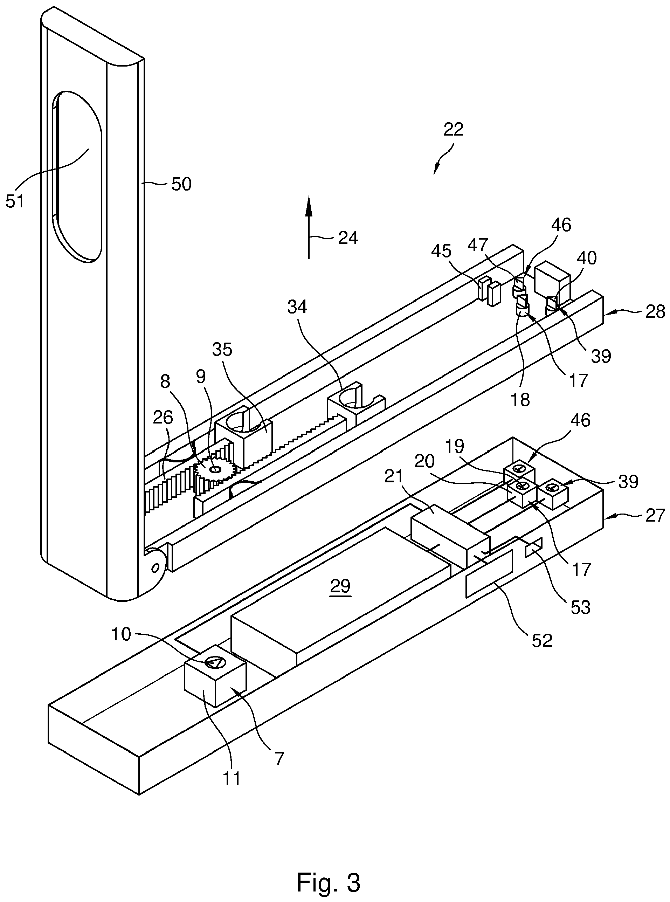

The plunger drive 7, the pump valve actuator 17 and the control unit 21 are part of a base unit 22 (shown separately in Fig. 3), while the pump housing 2, the plungers 3, 4, the supply conduit sections 12-15 and the delivery conduit 15 and the pump valve 16 are part of a disposable unit 23 (shown separately in Figs. 4-6) which is removably attached to the base unit 22. The coupling of the pump valve 16 to the pump valve actuator 17 and the coupling of the plungers 3, 4 to the plunger drive 7 are releasable.

This allows the parts of the pump 1 that are in direct contact with infusion liquid, in particular the pump cylinders and the pump valve, and which need to be sterile at least when perfusion is started, to be replaceable as a single disposable unit, which can be mounted and dismounted very easily. The rest of the pump 1, in particular the relatively costly plunger drive 7 and the valve actuators 17, 39, 40 can be re-used many times, so that costs are saved and relatively little waste is produced.

The base unit 22 has a lid 50 pivotably mounted to a main portion of the base unit 22. The lid 50 shields the mounted disposable unit 23, while a window 51 in the lid 50 allows visual inspection of the position of the plungers 3, 4 and, if the pump housing 2 is transparent at least between the window 51 and the chambers 5, 6, visual inspection of the liquid in the pump chambers 5, 6 in the housing 2.

In the present example, the base unit 22 has a bottom portion 27 and a top portion 28. In normal use, the bottom portion 27 remains attached to the top portion 28, so that the coupling member 10 remains connected to the shaft 9 and the pump valve motor coupling member 19 remains coupled to the pump valve coupling member 18. Only for repairs, such as exchanging a rechargeable battery 29, the bottom portion 27 and the top portion 28 of the base unit 22 are dismounted from each other.

For showing an operating condition of the pump 1, a display 52 connected to the control unit 21 is provided. For charging the battery 29 and for connecting the control unit 21 to an external device, a port 53 is provided, which may for instance be an USB-C port. This allows to display and control further features of operation and settings of the pump 1 via the external device. Data regarding operation and settings of the pump may also be monitored and/or registered by the external device, for instance to verify flow rates of liquid that have been administered to a patient during a given period of use. A transmitter/receiver for wireless communication between the control unit 21 and an external device may also be provided.

Removing a used disposable unit 23 from the base unit 22 and mounting a fresh disposable unit to the base unit 22 is made particularly easy, because the coupling of the pump valve 16 to the pump valve actuator 17 and the coupling of the plungers 3, 4 to the plunger drive 7 are releasable in one common direction 24. In the present example, this is achieved by providing couplings of the plungers 3, 4 to the toothed racks 25, 26 of the plunger drive 7 in the form of releasable ball joint connections 29, 30 of which outer members 34, 35 are open in the releasing direction 24 transverse to the direction in which plungers 3, 4 are movable, so that ball members 36, 37 can be moved out of engagement with the outer members 34, 35. Preferably, the opening of the outer members 34, 35 in the releasing direction 24 is slightly smaller than the contour of the ball members 36, 37, so that a small resistance has to be overcome to mount and dismount the ball joint connections 29, 30. Other releasable connections, such as T-connections or pin-hole connections, are also conceivable.

An advantage of ball joint connections 29, 30 is that during mounting, the disposable unit 23 can be oriented in a wide range of directions relative to the base unit 22 while the disposable unit 23 is connected to the base unit 22 only via one or both of the ball joint connections 29, 30. This facilitates mounting and dismounting of the disposable unit 23 and avoids damage to the coupling of the plungers 3, 4 to the plunger drive 7 if the disposable unit 23 is rotated relative to the base unit 22 while not fully disconnected at the coupling of the plungers 3, 4 to the plunger drive 7.

The supply conduit sections 12, 13 and the delivery conduit 15 have end portions 31-33 distally from the pump chambers 5, 6 that are arranged for connection to infusion transfer tubing. In this example, the end portions are provided in the form of projections 31-33 insertable into ends of the tubing for frictional engagement with the tubing ends that are slightly expanded by the ends 31-33 inserted therein.

The two upstream supply conduit sections 12, 13 in the disposable unit 23 are each provided with a connection 31, 32 for infusion transfer tubing and merge into a downstream section 14. A three-way source selector valve 38 is provided between the upstream supply conduit sections 12, 13 and the downstream supply conduit section 14 for selectively causing a first or a second one of the upstream supply conduit sections 12, 13 to communicate with the downstream supply conduit section 14. The base unit 22 is provided with a source selector valve actuator 39 with a coupling 40 releasably coupled to the source selector valve 38. Operating the source selector valve 38 allows switching between supply from a first source communicating with the supply conduit section 12 (see Fig. 6) and supply from a second source communicating with the supply conduit section 13 (see Fig. 5). The first source may for instance be an IV bag filled with standard IV solution while the second source may for instance be a container holding a liquid medicament. During filling of the pump chamber 5 or 6 by means of a given displacement of the plunger 3 or 4, supplying from the second source during a predetermined portion of that displacement of the plunger 3 or 4 allows to mix in a predetermined dosage volume or concentration of the medicament to be administered into the total volume of liquid supplied into the pump chamber. In the pump chamber 5 or 6, the medicament is then, at least to a certain extent, mixed with the IV solution and can subsequently be administered to the patient during delivery of the supplied liquid from the pump chamber 5 or 6 into which it has been supplied.

The source selector valve actuator 39 is also connected to the control unit 21 which is arranged for causing the source selector valve actuator 39 to be actuated in accordance with a dosage volume or mixing ratio setting and displacement of the plungers 3, 4 during filling of the respective pump chambers 5, 6.

Because the source selector valve 38 is also included in the disposable unit 23 and releasably coupled to the source selector valve actuator 39 of the base unit 22, also the coupling of the source selector valve 38 and the source selector valve actuator 39 is made and released as the disposable unit 23 is mounted or dismounted.

In the present example, the source selector valve 38 is a three-way valve that can be switched between a first operating position providing open communication between the first supply conduit section 12 and the common supply conduit section 14 while blocking supply from the second supply conduit section 13 to the common supply conduit section 14 and a second operating position providing open communication between the second supply conduit section 13 and the common supply conduit section 14 while blocking supply from the first supply conduit section 12 to the common supply conduit section 14. This allows controlling dosage volumes or concentration using a single valve. It is however also possible to provide separate valves for opening and closing the first and second supply conduit sections. Furthermore, it is also possible to provide three or more upstream supply conduit sections which associated tubing connections to allow mixing from three or more sources.

The disposable unit 23 is further provided with a three-way bleeding valve 41 in the delivery conduit 15, a bleeding conduit 42 branching off from the delivery conduit 15 at the bleeding valve 41, a bubble detector 43 and a bubble detector signal output port in the form of contact 44. The base unit 22 is further provided with a bubble detector signal input port in the form of contact 45 in communication with the bubble detector signal output port 44 and a bleeding valve actuator 46 with a coupling 47 releasably coupled to the bleeding valve 41. The control unit 21 is connected and arranged for operating bleeding valve actuator 46 for causing the bleeding valve 41 to switch, in response to a signal from the bubble detector 43 representing detection of presence of air in liquid being delivered from the pump chamber 5 or 6 through the delivery conduit 15, from a position in which the delivery conduit 15 is open until its downstream end to a position in which the bleeding conduit 42 communicates via the bleeding valve 41 with the portion of the delivery conduit 15 upstream of the bleeding valve 41 and the one of the pump chambers 5, 6 from which liquid is delivered. Thus, detected air is discharged via the bleeding conduit 42 together with a small amount of the liquid. If no further air is detected, first the piston 3 or 4 is displaced over a small further distance, to allow for displacement of the detected air from the bubble detector 43 until downstream of the bleeding valve 41, and then the bleeding valve 41 is switched back to the position shown in Fig. 6 for resuming delivery of liquid via the delivery conduit 15. Thus, bubbles can reliably be evacuated from the liquid to be administered.

In this example the bleeding conduit opens into the environment at a connection 64. However, a tube for discharging liquid spilled during bleeding or a receptacle or an absorbing body for receiving such spilled liquid may be provided to avoid spilling of liquid outside of the pump.

During priming of the pump chamber 5, the plunger 3 can first be moved outwardly with the pump valve in the position shown in Fig. 6, in which the pump chamber 5 communicates with one of the supply conduit sections 12 and 13 and with the common supply conduit section 14, so that the internal volume of the first pump chamber 5 is increased and liquid is supplied into the first pump chamber 5. Then, the plunger 3 is stopped and the pump valve 16 is switched into the position shown in Fig. 5, in which the pump chamber 5 communicates with the delivery conduit 15. With the bleeding valve 41 in the position shown in Fig. 5, in which the pump chamber 5 communicates with the bleeding conduit 42 and while holding the pump 1 in an orientation in which the transfer conduit 48 opens into an upper end of the pump chamber 5, the plunger 3 is subsequently moved inwardly, so that the internal volume of the pump chamber 5 is reduced and air and priming liquid are expelled through the bleeding conduit 42. The other pump chamber 6 can be primed in a corresponding manner.

Instead of by a bubble detection and diversion system, bubble evacuation may also be carried out passively, for instance as in a conventional drip chamber.

The valves 16, 38, 41 are rotary valves each having a plug rotatable in the pump housing 2, the plugs each have passages arranged for providing communication between an inlet one and an outlet one of ports in the pump housing that are facing the plug in selected rotary positions of the plug. The plugs have axes of rotation in the direction 24 in which the disposable part 23 is releasable from the base part 22. This allows the valves 16, 38 and 41 in the disposable unit 23 to be operated by and to be releasably coupled to the actuators 17, 39 and 46 in the base unit 22 in a simple manner. In the present example, all valves are three-way or four-way versions of such rotary valves. It is however also possible to provide the disposable unit with only one or any other number of such valves and to provide one or more of these valves with a different number of ports, e.g. just two ports, for instance in the form of a stopcock valve, or a larger number of ports, for instance for bleeding directly from the pump valve.

To ensure that the position of the valve actuators 17, 39 and 46 are associated to positions of the plugs of the valves 16, 38 and, respectively, 41 in a one on one relationship only, the plugs and the valve actuators 17, 39 and 46 coupled thereto have complementary shaped coupling faces, which are each non-rotation symmetrical about the axis of rotation of the respective plug. Thus, for each valve plug, there is only one relative rotational position in which the associated valve actuator coupling 18, 40 and 47 can be coupled thereto. In the present example, the valve actuator couplings 18, 40, 47 each have a triangular projection of which the center is offset from the axis of rotation of the respective valve actuator coupling 18, 40, 47 and the plugs have matching triangular recesses in which the triangular projections only fit closely if the orientations of the valve actuator couplings 18, 40, 47 match the orientations of the plugs. The triangular projections visually resemble arrows, which facilitates checking whether the orientations of the valve actuator couplings 18, 40, 47 match the orientations of the plugs before mounting a disposable unit 23 to a base unit 22.

Because the pump 1 according to the present example has two of the plungers 3, 4 and the pump housing 2 and the plungers 3, 4 bounds two pump chambers 5, 6 and both plungers 3, 4 are reciprocally movable inwardly and outwardly of the pump housing 2, thereby increasing and decreasing the volume of the respective pump chamber 5, 6 and the plunger drive 7 is arranged for simultaneously driving the plungers 3, 4 in opposite directions, a single drive is sufficient for driving two plungers for supplying IV liquid to one of the pump chambers 5, 6 and delivering IV liquid from the other one of the pump chambers 5, 6. It is however also possible to provide a pump according to the invention with only one plunger or to drive movement of two or more plungers using separate associated drives each for driving one or a subgroup of the plungers.

In the pump 1 according to the present example, the plungers 3, 4 are coupled to each other for simultaneous movement in mutually opposite directions only. Thus, supplying IV liquid to one of the pump chambers 5, 6 and simultaneously delivering IV liquid from the other one of the pump chambers 5, 6 is achieved in a simple manner.

Because the pump valve 16 is four way valve in which ports are arranged for alternatingly causing one of the pump chambers 5, 6 to communicate with the delivery conduit and causing the other one of the pump chambers 5, 6 to communicate with the supply conduit and vice versa, operation of a single valve is sufficient for switching between supplying liquid to a first one of the pump chambers 5, 6 while delivering liquid from a second one of the pump chambers 5, 6 to supplying liquid to the second one of the pump chambers 5, 6 while delivering liquid from the first one of the pump chambers 5, 6.

The pump valve 16 is arranged so that there is no direct communication between, on the one hand, the supply conduit sections 12 or 13 and 14 and, on the other hand, the delivery conduit 15 in any position of the pump valve 16. Thus, uncontrolled supply of IV liquid bypassing the pump chambers 5, 6 is avoided, even if for instance the disposable unit 22 is dismounted without disconnecting the patient from the pump 1 or pressure is exerted onto the IV bag (e.g. as a result of falling). This is particularly useful for avoiding inadvertent direct and undiluted administration of a medicament that has been mixed in.

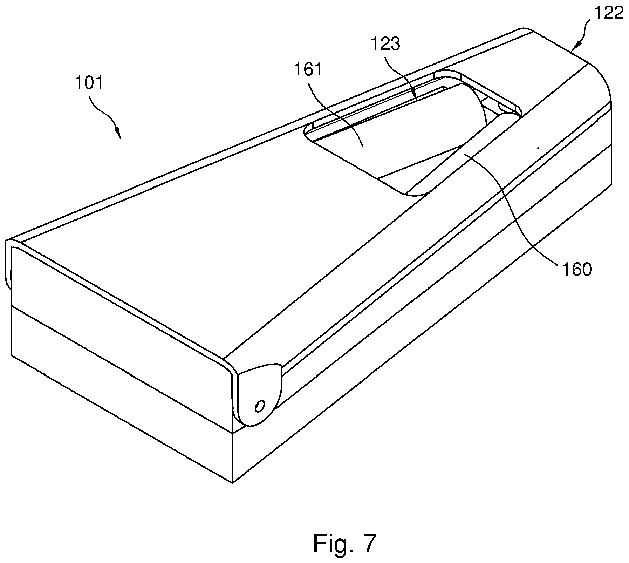

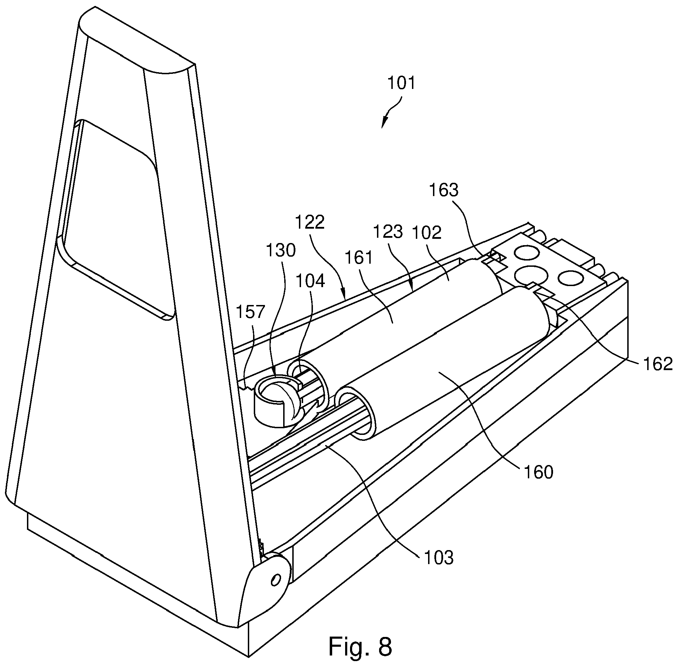

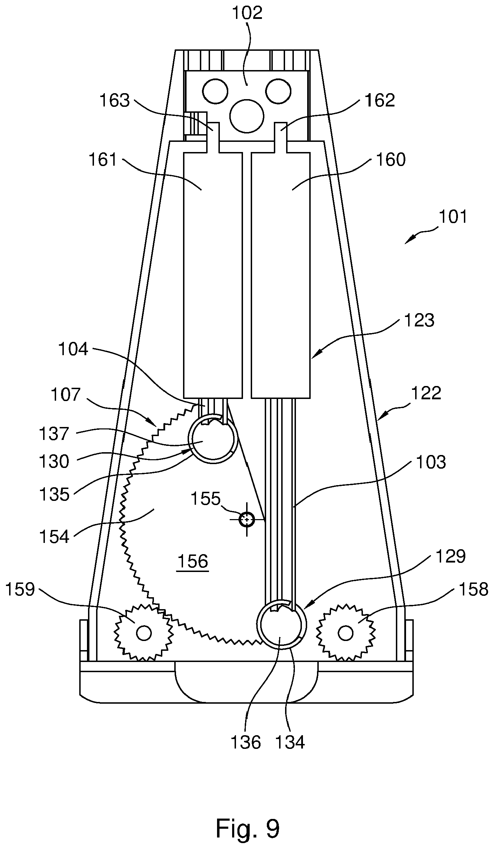

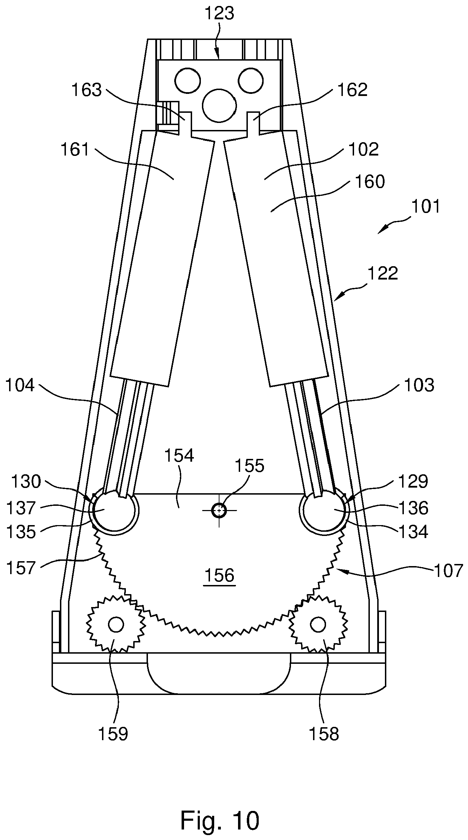

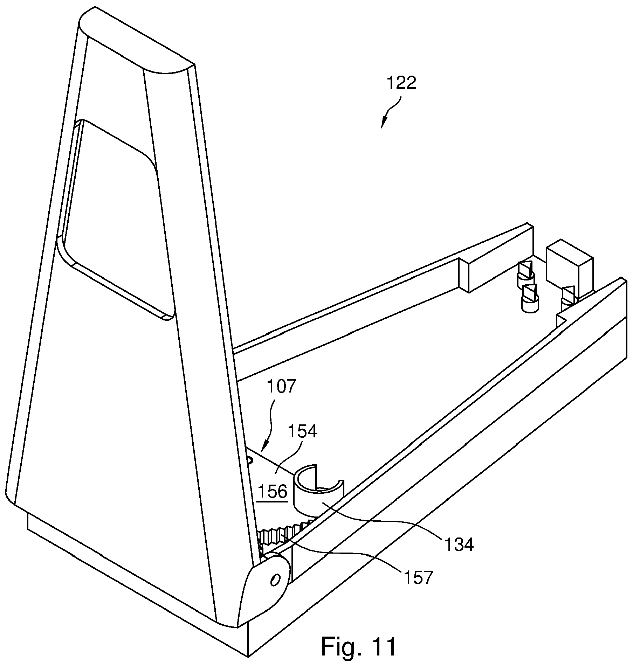

In Figs. 7-11, a second example of a pump 101 according to the invention is shown. Also this pump 101 is composed of a re-usable base unit 122 and a disposable unit 123. In Fig. 11, the re-usable base unit 122 is shown without the disposable unit 123 mounted thereto.

The plungers 103, 104 are coupled to a double arm 154 having an axis of rotation 155 extending centrally between the plungers 103, 104. Thus, the coupling of the plungers 103, 104 to each other is achieved in a very simple manner and the plungers 103, 104 are guided by the double arm 154 with very little friction.

The double arm 154 is a portion of a gear wheel 156 having a toothed gear rack 157 extending along at least a section of a circle concentric with the axis of rotation 155 of the double arm 154. The plunger drive 107 further has pinions 158, 159 coupled to an electric motor. The pinions 158, 159 drivingly engaging the toothed gear rack 157. Thus, pivoting of the double arm 154 about the axis of rotation, and thereby simultaneous movement of the plungers 103, 104 in opposite directions is driven in a simple manner. A particular advantage of driving movement of the plungers via a double arm is that the pump 101 can be relatively short in longitudinal direction, because of the absence of a toothed rack projecting from the plungers in the direction of movement of the plungers.

To allow the orientations of the pump chambers to accommodate to the movement of ends of the plungers 103, 104 along a circle segment, the pump housing 102 includes two pump housing parts 160, 161 each bounding one of the pump chambers, that are pivotably suspended near ends 162, 163 distally from the couplings 129, 130 engaging the plungers 103, 104.

Also in this example, the couplings 129, 130 of the plunger drive 107 to the plungers 103, 104 are provided in the form of ball joints of which the balls 136, 137 are releasable from outer coupling members 134, 135. In this example, a further advantage of the ball joints 129, 130 is that angular movement at the couplings 129, 130 during pivoting of the double arm 154 is also accommodated.

Several features have been described as part of the same or separate embodiments. However, it will be appreciated that the scope of the invention also includes embodiments having combinations of all or some of these features other than the specific combinations of features embodied in the examples.

## Claims

1. An infusion delivery pump comprising:
a pump housing;
a plunger, wherein the pump housing and the plunger bound a pump chamber, the plunger is reciprocally movable inwardly and outwardly of the pump housing thereby increasing and decreasing the volume of the pump chamber;
a plunger drive for driving displacement of the plunger relative to the pump housing, the plunger drive being coupled to the plunger;
a supply conduit communicating with the pump chamber for delivering infusion liquid from a reservoir, such as an IV bag, into the pump chamber;
a delivery conduit communicating with the pump chamber for receiving and delivering infusion liquid from the pump chamber;
at least one pump valve via which the supply conduit communicates with the pump chamber, while the delivery conduit is shut off from the pump chamber, if the pump valve is in an infusion intake condition and via which the delivery conduit communicates with the pump chamber, while the supply conduit is shut off from the pump chamber, if the pump valve is in an infusion delivery condition;
at least one pump valve actuator; and
a control unit arranged and connected for controlling the plunger drive and the pump valve actuator;
wherein the plunger drive, the pump valve actuator and the control unit are part of a base unit;
wherein the pump housing, the plunger, at least a downstream portion of the supply conduit and at least one upstream portion of the delivery conduit and the at least one pump valve are part of a disposable unit removably attached to the base unit, the coupling of the at least one pump valve to the at least one pump valve actuator and the coupling of the at least one plunger to the plunger drive being releasable.

2. A pump according to claim 1, wherein the coupling of the at least one pump valve to the at least one pump valve actuator and the coupling of the at least one plunger to the plunger drive are releasable in one common direction.

3. A pump according to claim 1 or 2, wherein the supply conduit and the delivery conduit have ends distally from the pump chamber that are arranged for connection of infusion transfer tubing.

4. A pump according to any one of the preceding claims, wherein, in the disposable unit, the supply conduit comprises at least two upstream supply conduit sections, each with a connection for infusion transfer tubing, and one downstream common supply conduit section, a source selector valve being provided for selectively causing a first or a second one of the upstream supply conduit sections to communicate with the common supply conduit section, and wherein the base unit further comprises a source selector valve actuator comprising a coupling releasably coupled to the source selector valve.

5. A pump according to claim any of the preceding claims, wherein the disposable unit further comprises a bleeding valve in the delivery conduit, a bleeding conduit branching off from the delivery conduit at the bleeding valve, a bubble detector and a bubble detector signal output port, and wherein the base unit further comprises a bubble detector signal input in communication with the bubble detector signal output port and a bleeding valve actuator releasably coupled to the bleeding valve, wherein the control unit is connected and arranged for operating the bleeding valve in response to a signal from the bubble detector representing detection of presence of air so that detected air is discharged via the bleeding conduit.

6. A pump according to any of the preceding claims, wherein at least one of the valves is a rotary valve with a plug rotatable in the pump housing, the plug having passages arranged for providing communication between an inlet one and an outlet one of ports facing the plug in at least one rotary position of the plug, the plug having an axis of rotation in a direction in which the disposable part is releasable from the base part.

7. A pump according to claim 6, wherein the plug and the valve actuator coupled thereto have complementary shaped coupling faces, which are non-rotation symmetrical about the axis of rotation of the plug.

8. A pump according to any of the preceding claims, comprising a further plunger, wherein the pump housing and the plunger bound a further pump chamber, the further plunger is reciprocally movable inwardly and outwardly of the pump housing thereby increasing and decreasing the volume of the further pump chamber and wherein the plunger drive is arranged for simultaneously driving the plungers in opposite directions.

9. A pump according to claim 8, wherein the plungers are coupled to each other for simultaneous opposite movement only, so that liquid is delivered from a pump chamber of one of the plungers only while liquid is supplied to a pump chamber of the other one of the plungers.

10. A pump according to claim 9, wherein the plungers are coupled to a double arm having an axis of rotation extending centrally between the plungers.

11. A pump according to claim 10, wherein the double arm is a portion of a gear wheel having a toothed gear rack extending along at least a section of a circle concentric with the axis of rotation of the double arm, the plunger drive further comprising a pinion drivingly engaging the toothed gear rack.

12. A pump according to claim 10 or 11, wherein the pump housing includes two pump housing parts each bounding one of the pump chambers, wherein the pump housing parts are pivotably suspended near ends distally from the couplings engaging the plungers.

13. A pump according to claim 6 or 7 and according to any of the claims 8-12, wherein the pump valve is four way valve in which ports are arranged for alteratingly causing one of the pump chambers to communicate with the delivery conduit and causing the other one of the pump chambers to communicate with the supply conduit and vice versa.

14. A pump according to any of the preceding claims, wherein the pump valve is arranged so that there is no direct communication between the supply conduit and the delivery conduit in any position of the pump valve.

15. A disposable unit for use in an infusion delivery pump, the disposable unit comprising:
a pump housing;
a plunger, wherein the pump housing and the plunger bound a pump chamber, the plunger is reciprocally movable inwardly and outwardly of the pump housing thereby increasing and decreasing the volume of the pump chamber;
a supply conduit communicating with the pump chamber for delivering infusion liquid from a reservoir, such as an IV bag, into the pump chamber;
a delivery conduit communicating with the pump chamber for receiving and delivering infusion liquid from the pump chamber; and
at least one pump valve via which the supply conduit communicates with the pump chamber, while the delivery conduit is shut off from the pump chamber, if the pump valve is in an infusion intake condition and via which the delivery conduit communicates with the pump chamber, while the supply conduit is shut off from the pump chamber, if the pump valve is in an infusion delivery condition;
wherein the at least one pump valve is arranged for releasable coupling to at least one pump valve actuator and the at least one plunger is arranged for releasable coupling to a plunger drive.
